**Europäisches Patentamt**

**European Patent Office**

**Office européen des brevets**

(19)

(11) Veröffentlichungsnummer : **0 309 883 B1**

(12)

# EUROPÄISCHE PATENTSCHRIFT

(45) Veröffentlichungstag der Patentschrift :
**23.11.94 Patentblatt 94/47**

(51) Int. Cl.$^5$ : **G01N 33/52,** // G01N33/72

(21) Anmeldenummer : **88115457.9**

(22) Anmeldetag : **21.09.88**

(54) Testträger zur analytischen Bestimmung eines Bestandteils einer Körperflüssigkeit.

(30) Priorität : **30.09.87 DE 3733084**

(43) Veröffentlichungstag der Anmeldung :
**05.04.89 Patentblatt 89/14**

(45) Bekanntmachung des Hinweises auf die
Patenterteilung :
**23.11.94 Patentblatt 94/47**

(84) Benannte Vertragsstaaten :
**AT BE CH DE ES FR GB GR IT LI LU NL SE**

(56) Entgegenhaltungen :
EP-A- 0 045 476
EP-A- 0 208 952
EP-A- 0 271 854
DE-A- 3 130 749
DE-A- 3 323 973

(73) Patentinhaber : **BOEHRINGER MANNHEIM
GMBH
D-68298 Mannheim (DE)**

(72) Erfinder : **Freitag, Helmut, Dr.
9115 Hague Road
Indianapolis, IN 46250 (US)**
Erfinder : **Rothe, Anselm, Dr.
Tiefenklingerweg 21
D-6943 Birkenau (DE)**
Erfinder : **Pollmann, Klaus, Dr.
9115 Hague Road
Indianapolis, IN 46250 (US)**

## Beschreibung

Die Erfindung betrifft einen Testträger zur analytischen Bestimmung eines Bestandteils einer flüssigen Probe, insbesondere einer Körperflüssigkeit, mit einer Tragschicht, die teilweise von einer flüssigkeitsabsorbierenden Schicht, welche eine Probenauftragszone und eine Transportzone beinhaltet, bedeckt ist und einer ersten ersten Reagenzschicht, die mit der flüssigkeitsabsorbierenden Schicht dauerhaft in Verbindung steht sowie einer oder mehreren weiteren Reagenzschichten, die durch externe Manipulation mit der flüssigkeitsabsorbierenden Schicht in Verbindung gebracht werden können sowie die Verwendung eines solchen Testträgers zur Bestimmung eines Bestandteils einer flüssigen Probe, insbesondere einer Körperflüssigkeit.

Testträger haben bei der Analyse von Flüssigkeiten eine erhebliche Bedeutung erlangt. Insbesondere in der klinischen Chemie, die sich mit der qualitativen und quantitativen analytischen Bestimmung von Bestandteilen von Körperflüssigkeiten (insbesondere Blut und Urin) befaßt, werden ihre Vorteile sehr geschätzt. Während die klassischen, mit flüssigen Reagenzien arbeitenden Methoden eine Vielzahl von Handhabungsschritten erfordern, zeichnen sich analytische Bestimmungen mit Hilfe von Testträgern durch eine äußerst einfache Handhabung aus. Bei der Analyse von Urin werden im allgemeinen Testträger in Form von Teststreifen eingesetzt, die in die Probe kurz eingetaucht und danach entweder visuell oder apparativ ausgewertet werden. Bei der Blutanalyse wird ein Tropfen Serum oder - bei einer besonders fortschrittlichen Art von Testträgern - Blut an einer vorbestimmten Stelle des Testträgers aufgegeben.

Die Reaktion der Flüssigkeit mit den auf dem Testträger vorhandenen Reagenzien führt zu einer wahrnehmbaren Veränderung des Testträgers, meist zu einer Veränderung seiner Farbe, z. B. durch Bildung oder Freisetzung eines Farbstoffes. Die Reaktion kann aber auch zur Entstehung oder Änderung einer fluoreszierenden oder in anderer Weise optisch oder in sonstiger Weise wahrnehmbaren Substanz führen. Testträger für die Bestimmung von Blutbestandteilen werden überwiegend apparativ mit Hilfe entsprechender Geräte ausgewertet. Die vorliegende Erfindung richtet sich insbesondere auf Testträger zur quantitativen Bestimmung von Flüssigkeitsbestandteilen mit Hilfe eines Reflexionsphotometers oder eines anderen optischen Auswertegerätes.

In der Anfangszeit der Entwicklung von Testträgern besaßen diese nur eine einzige Testschicht, auf der verschiedene Reagenzien derartig kombiniert waren, daß auch komplizierte Reaktionsfolgen möglich waren. Einschichtige Testträger lassen jedoch kein zeitlich definiertes Ablaufen mehrerer hintereinander geschalteter Reaktionen zu, was eine zuverlässige quantitative Bestimmung eines Analyten mittels kinetischer Messung unmöglich macht. Außerdem ist die Stabilität von Mischungen unterschiedlicher Reagenzien gegenüber den reinen Stoffen oft beträchtlich herabgesetzt, was ebenfalls letztendlich leicht zu unzuverlässigen Meßwerten führen kann.

Dies hat zur Entwicklung von Testträgern geführt, bei denen Reagenzien in unterschiedlichen Reagenzschichten vorliegen und diese außerdem so auf einer Tragschicht angeordnet sein können, daß ein zeitlich definiertes Ablaufen hintereinander geschalteter Reaktionen möglich ist.

In DE-OS 31 30 749 werden Testträger zur Bestimmung verschiedener Parameter in Serum, Plasma oder Vollblut beschrieben, bei denen die Tragschicht durch eine längliche Basisfolie gebildet wird. Darauf ist eine flüssigkeitsabsorbierende Schicht angeordnet. An der Basisfolie ist eine rechteckige Deckfolie, die ebenso breit ist wie die Basisfolie, aber erheblich kürzer als diese, mit einer Kante befestigt. Die Befestigungsstelle liegt in der Nähe des Endes der flüssigkeitsabsorbierenden Schicht. Auf der der Basisfolie zugewandten Seite der Deckfolie befindet sich eine Reagenzschicht, insbesondere ein Reagenzfilm. Die Deckfolie mit dem Reagenzfilm bildet eine Klappe, wobei die Befestigung an der Basisfolie so gestaltet ist, daß die Klappe im Ruhezustand die flüssigkeitsabsorbierende Schicht nicht berührt, durch externe Manipulation aber mit ihr in Verbindung gebracht werden kann. Dadurch ist es möglich, den zeitlichen Ablauf einer Bestimmungsreaktion in Schritte zu unterteilen. Die Probe wird hierfür zunächst in einer Probenauftragszone auf die flüssigkeitsabsorbierende Schicht aufgebracht, die im Falle, daß Vollblut als Probe verwendet wird, so beschaffen sein muß, daß die Erythrozyten abgetrennt und zurückgehalten werden, während die Probe in die sich anschließende Transportzone der flüssigkeitsabsorbierenden Schicht gesaugt wird. Die Probe kann dann, falls dies z. B. für eine enzymatische Bestimmung notwendig ist, innerhalb der flüssigkeitsabsorbierenden Schicht temperiert werden. Zu einem bestimmten Zeitpunkt wird die Deck-folie mit ihrer Reagenzschicht auf die Basisfolie aufgedrückt und damit mit der flüssigkeitsabsorbierenden Schicht in Verbindung gebracht. Durch diesen vollflächigen Kontakt kann die in der flüssigkeitsabsorbierenden Schicht enthaltene Probe in die Reagenzschicht der Klappe übertreten und dort zu einem bestimmten Zeitpunkt eine Reaktion auslösen, die zu einem nachweisbaren Signal, beispielsweise zu einem Farbumschlag führt.

Ein wesentlicher Nachteil solcher Testträger besteht darin, daß keine zeitlich entkoppelten Reaktionen im Sinne von chemischen, enzymatischen oder immunologischen Reaktionsschritten einer Reaktionsfolge einer zum Nachweis eines Inhaltsstoffes einer Körperflüssigkeit erforderlichen Gesamtreaktionssequenz durchgeführt werden können. Sobald die auf der Klappe angeordnete Reagenzschicht die sämtliche zum Nachweis erforderlichen Bestandteile enthält, mit der in der flüssigkeitsabsorbierenden Schicht verteilten Probe in Verbindung gebracht wird, läuft die Gesamtreaktionssequenz ohne definierte zeitliche Unterbrechungsmöglichkeit ab. Auch eine Konditionierung der Probe, d. h. eine derartige chemische, enzymatische oder immunologische Aufbereitung, daß die Nachweisreaktion anschließend schneller, vollständiger oder besser meßbar ablaufen kann, ist so nicht möglich.

Zwar können die für die Gesamtreaktionssequenz oder die Konditionierung benötigten Reagenzien je nach entsprechenden Teilreaktionen auf mehrere Reagenzschichten, die unabhängig voneinander nacheinander auf die Basisfolie gedrückt werden können, aufgeteilt werden, eine apparative Durchführung eines solchen Prinzips zur Bestimmung eines Analyten in einer Körperflüssigkeit erweist sich jedoch als äußerst aufwendig und umständlich. Darüber hinaus benötigt jede Reagenzschicht zur Benetzung ein bestimmtes Volumen an Flüssigkeit. Je mehr Reagenzschichten eingesetzt werden, desto mehr Probenvolumen ist zur Benetzung aller Schichten erforderlich. Dies ist ein erheblicher Nachteil, da gerade danach getrachtet wird, von Körperflüssigkeiten, wie z. B. Blut, immer nur möglichst geringe Mengen für Untersuchungen einzusetzen.

Zeitlich entkoppelte Reaktionen sind bei den beschriebenen Testträgern der DE-OS 31 30 749 prinzipiell auch durch Imprägnierung der flüssigkeitsabsorbierenden Schicht mit entsprechenden löslichen Reagenzien möglich. Diese Möglichkeit leidet aber unter dem Mangel, daß sich aufgrund des Flüssigkeitstransports innerhalb der flüssigkeitsabsorbierenden Schicht imprägnierte Reagenzien in der Flüssigkeitsfront anreichern und Chromatographie-Effekte auftreten können, die sich in der Ausbildung räumlicher Konzentrationsgradienten und letztendlich in nicht reproduzierbaren und verfälschten Meßergebnissen auswirken können. Auch ist nicht jedes Material für die Imprägnierung mit Reagenzien geeignet.

Da außerdem die flüssigkeitsabsorbierende Schicht im Falle des Einsatzes von Vollblut auch mit Erythrozyten in Kontakt kommt, können solche Reagenzien, die mit Erythrozyten so wechselwirken, daß sie dadurch die Nachweisreaktion stören können, nicht zur Imprägnierung dieser Schicht verwendet werden. So hat z. B. der Einsatz von Stoffen zur Imprägnierung der flüssigkeitsabsorbierenden Schicht, die hämolytisch wirken, ein Auslaufen der Inhaltsstoffe der roten Blutkörperchen zur Folge, die beispielsweise durch ihre Eigenfarbe oder durch ihre chemische, enzymatische oder immunologische Reaktivität den Test erheblich stören können. Unter Umständen, z. B. im Falle der Bestimmung von Bilirubin in Vollblut werden so aufgrund der Freisetzung von Hämoglobin aus den Erythrozyten völlig falsche Meßwerte erhalten.

Es sind auch ähnliche Testträger bekannt, die mit Reagenzien imprägnierte Schichten in der Probenauftragszone, über der flüssigkeitsabsorbierenden Schicht aber in saugfähigem Kontakt mit dieser und eventuell unter einer Erythrozytenabtrennschicht, wie sie z.B. in DE-OS 30 29 579 oder DE-OS 33 23 973 beschrieben ist, enthalten. Hier kommen ebenfalls bei Probenaufgabe die in der Reagenzschicht imprägnierten Stoffe mit Erythrozyten in Kontakt und Produkte, die durch diese Wechselwirkung entstehen, können in die Transportzone der flüssigkeitsabsorbierenden Schicht gelangen, wo sie wie bereits ausgeführt dann erheblich stören können. Es gelten deshalb die selben Einschränkungen für die zu verwendenden Reagenzien wie vorstehend beschrieben.

Im Falle der zuletzt beschriebenen Testträger stellt es sich außerdem als Nachteil heraus, daß je nach Art und Weise der Probenaufgabe auf die Probenauftragszone, die dort angebrachten Reagenzschichten in unterschiedlichem Ausmaß von der Probe benetzt werden können. So kann z. B. bei gegebener Größe der Probenaufgabezone das gleiche Probenvolumen so aufgegeben werden, daß nur eine kleine Fläche dieser Schichten mit der Probe in Berührung kommt. Es ist aber auch möglich, die Probenaufgabe so durchzuführen, daß die Reagenzschichten vollständig von der Probe benetzt werden. Das Ausmaß der Benetzung hängt deshalb bei diesen Testträgern mit von der Geschicklichkeit des Auftragenden ab. Damit ist auch die Konzentration des Reagenzes, das sich bei Benetzung der in der Probenauftragszone befindlichen Reagenzschicht in der Probe löst, von diesem Parameter abhängig, was letztendlich dann, wenn es auf die Kontrolle exakt einzustellender Konzentrationen dieses Reagenzes in der Probe ankommt, zu ungenauen, stark streuenden Meßergebnissen oder auch völlig verfälschten Resultaten führen kann.

EP-A 208952 beschreibt einen Testträger mit einer Aufgabezone, einer flüssigkeitsabsorbierenden Schicht als Transportzone, einer Reagenzschicht außerhalb der Aufgabezone, die durch Annähen auf der Oberfläche der flüssigkeitsabsorbierenden Schicht aufgebracht ist und einer zusätzlichen Reagenzschicht, die durch externe Manipulation auf die erste Reagenzschicht aufgedrückt werden kann. Die Nähte, mit denen die Reagenzschicht auf die flüssigkeitsabsorbierende Schicht aufgenäht ist, werden als wesentlich für den ansonsten unbefriedigenden Flüssigkeitstransport in diese aufliegende Reagenzschicht beschrieben.

EP-A 271854 ist Stand der Technik nach Artikel 54 (3) EPÜ und beschreibt einen Testträger mit einer Aufgabenzone, einer flüssigkeitsabsorbierenden Schicht als Transportzone, einer daran anschließenden Saugzone und einer oder mehrerer auf der flüssigkeitsabsorbierenden Schicht außerhalb der Aufgabenzone aufgebrachten Reagenzschichten.

Es besteht ein Bedarf an Testträgern, die mit einer geringen Probenmenge auskommen, die Analyse von Blut ohne die Gefahr der Wechselwirkung mit Erythrozyten, z. B. Hämolyse, ermöglichen, bei denen die Konzentration an Reagenzien in der Probe kontrolliert und exakt eingestellt werden kann und mit denen Reaktionen zeitlich entkoppelt werden können.

Überraschenderweise wird dieses Bedürfnis bei einem Testträger der eingangs bezeichneten Art dadurch gelöst, daß die mit der flüssigkeitsabsorbierenden Schicht dauerhaft in Verbindung stehende mit einem Reagenz imprägnierte Schicht zwischen flüssigkeitsabsorbierender Schicht und Transportschicht angeordnet ist, an die Tragschicht fixiert ist, nicht mit der Probenauftragszone überlappt und sie vollflächig und dauerhaft mit der flüssigkeitsabsorbierenden Schicht in Verbindung steht und daß mindestens eine weitere Reagenzschicht, die durch externe Manipulation mit der flüssigkeitsabsorbierenden Schicht in Verbindung gebracht werden kann und die die dauerhaft mit der flüssigkeitsabsorbierenden Schicht in Verbindung stehende Reagenzschicht überdecken kann.

Als Probenauftragszone wird in diesem Zusammenhang der gesamte Bereich eines Testträgers und nicht nur die Aufgabeschicht verstanden, wo die Probe aufgegeben wird. Speziell dann, wenn es sich bei der Probe um Vollblut handelt, ist hierunter der gesamte Bereich zu verstehen, der nach dem Aufgeben der Probe Erythrozyten enthält.

Als Transportzone wird der gesamte Teil der von der flüssigkeitsabsorbierenden Schicht bedeckten Tragschicht bezeichnet, der nicht zur Probenauftragszone gehört.

Eine Überlappung von Probenauftragszone und mit der flüssigkeitsabsorbierenden Schicht dauerhaft in Verbindung stehenden Reagenzschicht liegt immer dann vor, wenn Teile der Schicht in die Zone hineinragen und so eine quasi flächige Berührung stattfindet. Im Sinne der vorliegenden Erfindung wird aber auch dann von einer Überlappung gesprochen, wenn Probenauftragszone und Reagenzschicht aneinanderstoßen und sich quasi nur mit ihren äußeren Begrenzungen oder Kanten berühren. Erfindungsgemäß sind Probenauftragszone und mit der flüssigkeitsabsorbierenden Schicht in Verbindung stehende Reagenzschicht räumlich getrennt angeordnet.

Im Rahmen der vorliegenden Erfindung stehen flüssigkeitsabsorbierende Schicht und die in der Transportzone des Testträgers befindliche Reagenzschicht dauerhaft miteinander in Verbindung. Dies bedeutet jedoch nicht, daß sie in direktem unmittelbaren Kontakt zueinander stehen müssen. Auch ein mittelbarer Kontakt, z. B. durch eine oder mehrere Schichten, die einen Flüssigkeitsaustausch zulassen, soll nicht ausgeschlossen werden. Erfindungsgemäß wird allerdings der direkte Kontakt von flüssigkeitsabsorbierender Schicht und damit in Verbindung stehender Reagenzschicht bevorzugt.

Entsprechendes gilt auch für eine Reagenzschicht des Testträgers, die durch externe Manipulation mit der flüssigkeitsabsorbierenden Schicht in Verbindung gebracht werden kann. Auch dort kann nach Herstellung der Verbindung zwischen Reagenzschicht und flüssigkeitsabsorbierender Schicht ein direkter unmittelbarer Kontakt hergestellt worden sein. Es ist aber auch möglich, daß beide Schichten dann über eine oder mehrere andere Schichten, die einen Flüssigkeitsaustausch zulassen, miteinander in Verbindung stehen.

Auf jeden Fall muß im erfindungsgemäßen Testträger die mit der flüssigkeitsabsorbierenden Schicht in Verbindung stehende Reagenzschicht so angeordnet sein, daß beide so übereinander liegen, daß sie durch einen in Bezug auf die flüssigkeitsabsorbierende Schicht vertikalen Flüssigkeitsaustausch miteinander in Verbindung stehen.

Gleiches gilt auch für einen Testträger mit dem zeitlich entkoppelte Reaktionen durchgeführt werden sollen. Dort müssen die mit der flüssigkeitsabsorbierenden Schicht dauerhaft in Verbindung stehende Reagenzschicht und die durch externe Manipulation mit der flüssigkeitsabsorbierenden Schicht in Verbindung zu bringende Reagenzschicht so angeordnet sein, daß beide nach externer Manipulation so übereinander zu liegen kommen, daß beide durch einen in Bezug auf die flüssigkeitsabsorbierende Schicht vertikalen Flüssigkeitsaustausch miteinander in Verbindung stehen. In der Regel wird dies durch Andrücken der in Verbindung mit der flüssigkeitsabsorbierenden Schicht zu bringenden Schicht in Richtung auf die Tragschicht erreicht.

Der erfindungsgemäße Testträger unterscheidet sich in seinem Aufbau von bekannten Testträgern dadurch, daß eine mit der flüssigkeitsabsorbierenden Schicht dauerhaft in Verbindung stehende Reagenzschicht so angeordnet ist, daß sie nicht mit der Probenauftragszone in direktem unmittelbarem Kontakt steht. Ein Flüssigkeitsaustausch ist aber natürlich möglich. In der Probenauftragszone zurückgehaltene Probenbestandteile kommen mit der Reagenzschicht so nicht in Berührung.

In einem bevorzugten erfindungsgemäßen Testträger ist eine mit der flüssigkeitsabsorbierenden Schicht dauerhaft in Verbindung stehende Reagenzschicht so in das von der Probenauftragszone entfernte Ende der Transportzone eingefügt, daß innerhalb der Transportzone eine Richtungsänderung des nach Probenaufgabe durch die flüssigkeitsabsorbierende Schicht erzeugten Flüssigkeitsstromes erzeugt wird. Besonders bevorzugt ist eine Änderung des Flüssigkeitsstromes in vertikaler Richtung zur flüssigkeitsabsorbierenden Schicht. Eine solche Änderung des Flüssigkeitsstromes wird erfindungsgemäß dadurch erzeugt, daß die mit der flüs-

4

sigkeitsabsorbierenden Schicht in Verbindung stehende Reagenzschicht aus saugfähigem Material besteht und innerhalb der Transportzone vollflächig, nicht aber nur über Kanten mit der flüssigkeitsabsorbierenden Schicht Flüssigkeit austauschen kann.

Die Reagenzschicht ist außerhalb der Probenauftragszone zwischen Tragschicht und flüssigkeitsabsorbierender Schicht angeordnet. Sie ist dabei an der Tragschicht fixiert, bevorzugt mit einem Schmelzkleberstreifen, so daß sie in ihrer Lage gehalten wird und nicht verrutschen kann.

Die flüssigkeitsabsorbierende Schicht kann aus einer Vielzahl von Materialien bestehen. Besonders geeignet sind Papiere, Vliese und solche Filmschichten, die aufgrund einer offenen, zahlreiche Kapillarspalten aufweisenden Struktur ein erhebliches Aufnahmevermögen für Flüssigkeit haben. Dieses Aufnahmevermögen für Flüssigkeit muß so groß sein, daß die darin absorbierte Flüssigkeitsmenge ausreicht, um sämtliche für die Nachweisreaktion erforderlichen Reagenzschichten vollständig zu benetzen.

Die im erfindungsgemäßen Testträger zum Einsatz kommenden Reagenzschichten bestehen im allgemeinen aus mit Reagenzien imprägnierten Materialien, insbesondere Geweben, die saugfähige Eigenschaften haben. Insbesondere durch die Anordnung der mit der flüssigkeitsabsorbierenden Schicht in Verbindung stehenden Reagenzschicht an dem der Probenauftragszone entfernten Ende der Transportzone wird so ein guter und gleichmäßiger Flüssigkeitsaustausch mit der flüssigkeitsabsorbierenden Schicht ermöglicht, was innerhalb relativ kurzer Zeit zu einer homogenen Lösung innerhalb dieser Zone des erfindungsgemäßen Testträgers führt. Es können auch wasserlösliche Filme zum Einsatz kommen, die die erforderlichen Reagenzien enthalten und die bei Kontakt mit der Probe gelöst werden. Solche Filme können aus hochmolekularem, polymeren wasserlöslichen Material bestehen. Besonders geeignet als Filmbildner sind solche Materialien, die auch als Binde- oder Quellmittel oder als Verdicker eingesetzt werden, wie z. B. Xanthan-Gum.

Aufgrund der speziellen Anordnung weist der erfindungsgemäße Testträger überraschende Vorteile gegenüber den aus dem Stand der Technik bekannten Testträgern auf. So ist die Konzentration des von der mit der flüssigkeitsabsorbierenden Schicht in Verbindung stehenden Reagenzschicht gelösten Reagenzes in der Probe unabhängig von der Art der Probenaufgabe auf die Probenauftragszone und damit exakt und kontrolliert einstellbar. Dies ist insbesondere für solche Tests wichtig, in denen es entscheidend auf die genaue Kontrolle eingesetzter Reagenzien z. B. auch in zeitlich entkoppelten Reaktionen ankommt.

Überraschenderweise wird gerade durch die erfindungsgemäße Anordnung des Testträgers bereits innerhalb sehr kurzer Zeit eine homogene Lösung innerhalb der Transportzone erhalten. Chromatographie-Effekte treten aufgrund der vollständigen und gleichmäßigen Benetzung der mit der flüssigkeitsabsorbierenden Schicht in Verbindung stehenden Reagenzschicht mit Probenflüssigkeit nicht auf.

Da Reagenzien dieser Reagenzschicht z. B. bei Einsatz von Vollblut nicht mit Erythrozyten in Berührung kommen, können auch solche Stoffe für Teste eingesetzt werden, die mit Erythrozyten den Test störende Wechselwirkungen eingehen könnten. So ist z. B. mit dem erfindungsgemäßen Testträger der Einsatz hämolytisch wirkender Stoffe, wie z. B. Dyphyllin in der mit der flüssigkeitsabsorbierenden Schicht in Verbindung stehenden Reagenzschicht möglich. Dies ist insbesondere für Teste auf Bilirubin aus Vollblut sehr wichtig, da hier Hämolyse und damit der Austritt von Inhaltsstoffen roter Blutkörperchen zu völlig verfälschten Resultaten führt. Solche Teste sind besonders empfindlich gegen Hämolyse, können jedoch mit dem erfindungsgemäßen Testträger in ausgezeichneter und reproduzierbarer Genauigkeit durchgeführt werden.

Während es bisher aus dem Stand der Tecknik bekannt war, daß jede zusätzliche Schicht in einem Testträger mehr Probenvolumen benötigt, trifft dies für den erfindungsgemäßen Testträger nicht zu. Im Gegenteil trägt hier die mit der flüssigkeitsabsorbierenden Schicht in Verbindung stehende Reagenzschicht aufgrund ihrer saugfähigen Eigenschaften dazu bei, die Flüssigkeit mehr aus der Probenauftragszone abzuziehen und an dem anderen Ende der flüssigkeitsabsorbierenden Schicht anzusammeln, so daß ausreichend Flüssigkeit zur Benetzung der mit dieser durch externe Manipulation in Verbindung zu bringenden Reagenzschicht zur Verfügung steht.

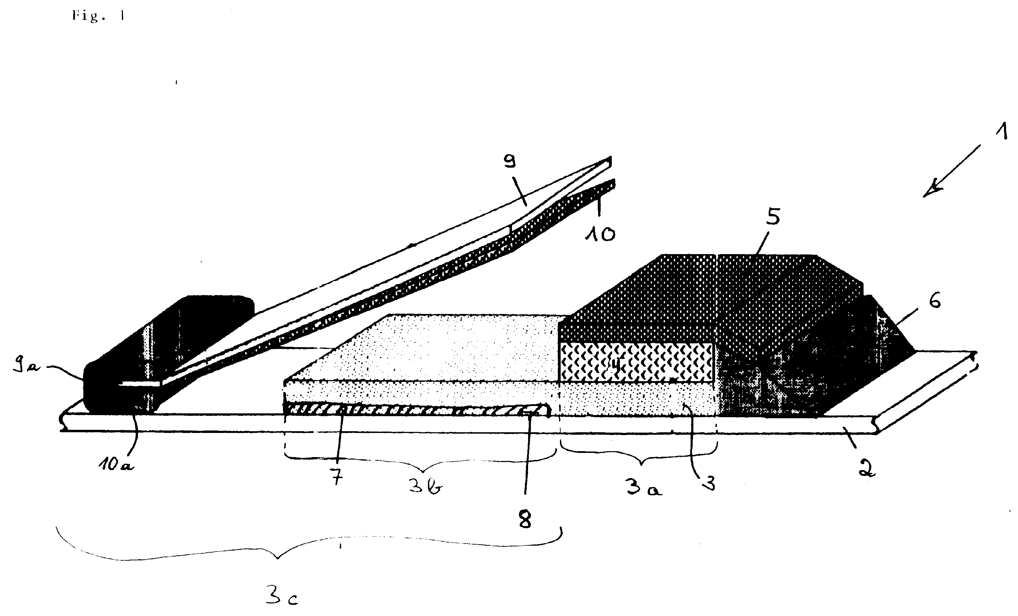

Die Erfindung wird im folgenden anhand eines in Fig. 1 schematisch dargestellten Ausführungsbeispiels näher erläutert.

Der in Fig. 1 dargestellte Testträger 1 hat eine Basisfolie 2, die als Tragschicht für eine vollflächig anliegende flüssigkeitsabsorbierende Schicht 3 dient. Die Basisfolie 2 ist schmal und langgestreckt wie bei einem Teststreifen. Die darauf angeordneten Schichten haben die gleiche Breite wie die Basisfolie 2, erstrecken sich aber nur über einen Teil von deren Länge.

Der gesamte Testbereich des Testträgers 1 läßt sich in eine Probenauftragszone 3 a und in einen Auswertungsbereich 3 c unterteilen. Innerhalb des Auswertungsbereichs 3 c liegt auch die Transportzone 3 b der flüssigkeitsabsorbierenden Schicht 3. Die Transportzone 3 b umfaßt den Bereich der flüssigkeitsabsorbierenden Schicht 3, der nicht zur Probenauftragszone 3 a gehört.

In der Probenauftragszone 3 a ist oberhalb der flüssigkeitsabsorbierenden Schicht 3 eine Plasmaabtrennschicht 4 vorgesehen, die zum Schutz vor Beschädigungen mit einem Abdecknetz 5 überdeckt ist. Die Plasmaabtrennschicht 4 und das Abdecknetz 5 bedecken nur einen Teil der flüssigkeitsabsorbierenden Schicht. Sowohl die flüssigkeitsabsorbierende Schicht 3 wie auch die Plasmaabtrennschicht 4 und das Abdecknetz 5 sind mit einem Ende durch den Schmelzkleberstreifen 6 an die Basisfolie 2 fixiert.

In der Transportzone 3 b ist unter der flüssigkeitsabsorbierenden Schicht 3 eine Reagenzschicht 7 mit einem Schmelzkleberstreifen 8 auf der Basisfolie 2 fixiert.

Im Auswertungsbereich hat der Testträger eine insgesamt mit 9 bezeichnete Klappe und einen Reagenzträger 10. Die Klappe 9 und der Reagenzträger 10 sind jeweils rechteckig und haben die Breite der Basisfolie 2. Mit einer ihrer kurzen Kanten 9 a bzw. 10 a sind sie derartig an der Basisfolie 2 befestigt, daß sie im entspannten Zustand nicht in Verbindung mit der flüssigkeitsabsorbierenden Schicht 3 stehen, jedoch durch externe Manipulation, z. B. Druck mit dieser in Verbindung gebracht werden können.

Die Klappe 9 besteht aus einer Deckfolie, die bevorzugt aus einem für sichtbares Licht durchlässigen Kunststoff besteht. Besonders bevorzugtes Material hierfür ist z. B. Polycarbonat-Folie.

Zur Durchführung einer Analyse werden beispielsweise 30 µl Blut auf das Abdecknetz 5 und die Plasmaabtrennschicht 4 aufgebracht. Die Probe sickert durch das Kunststoffnetz und die Plasmaabtrennschicht 4 in die flüssigkeitsabsorbierende Schicht 3, wobei die Erythrozyten im Blut abgetrennt werden (Näheres ist der DE-OS 30 29 579 zu entnehmen). In der flüssigkeitsabsorbierenden Schicht 3 wird das so gebildete Plasma von dieser in Längsrichtung des Testträgers in den Auswertungsbereich 3 c transportiert. Zu diesem Zweck hat die flüssigkeitsabsorbierende Schicht 3 Kapillartransporteigenschaften in dieser Richtung, also in Richtung ihrer Flächenausdehnung.

Die flüssigkeitsabsorbierende Schicht 3 und die Plasmaabtrennschicht 4 sind bevorzugt aus Glasfasern hergestellt (vgl. DE-OS 30 29 579). Sie können jedoch auch aus einem anderen Material, das die oben angegebenen Bedingungen erfüllt, hergestellt sein.

Nachdem die flüssigkeitsabsorbierende Schicht 3 schnell mit Plasma gefüllt ist, gelangt die Probe unter Änderung der bisherigen Flüssigkeitstransportrichtung durch Diffusion in die Reagenzschicht 7, wo eine Umsetzung der Probe mit dem auf die Schicht imprägnierten Reagenz stattfindet. Aufgrund der kurzen Diffusionswegstrecken hat sich bereits innerhalb kurzer Zeit, größenordnungsmäßig innerhalb einer Minute, eine homogene Lösung gebildet.

Die Reagenzschicht 7 hat bevorzugt eine für Flüssigkeiten offene Verbundstruktur und kann aus verschiedensten saugfähigen Materialien hergestellt sein, wie Kunststoff oder natürlichen Polymeren. Bevorzugt sind von möglichen Kunststoffmaterialien Polyamid- und besonders bevorzugt Polyestergewebe. Von natürlichen Materialien wird insbesondere Papier bevorzugt. Ganz besonders bevorzugtes Material für die Reagenzschicht 7 ist Teebeutelpapier, wie z. B. solches mit einem Flächengewicht von etwa 10 - 15 g/m$^2$, bevorzugt 12 g/m$^2$ aus Manila Langfaser-Hanf.

Nach Ablauf einer vorgegebenen Zeitspanne ab Probenaufgabe wird zu einem genau definierten Zeitpunkt durch Herunterdrücken der Klappe 9 auf die flüssigkeitsabsorbierende Schicht 3 eine weitere Reaktion eingeleitet. Aufgrund des Herunterdrückens der Klappe 9 wird der Reagenzträger 10 mit dem mit dem in Reagenzschicht 7 imprägnierten Reagenz umgesetzten Plasma in Verbindung gebracht, wodurch dieses unter erneuter Änderung der Flüssigkeitstransportrichtung in den Träger gesaugt und dort weiter zur Reaktion gebracht wird, was letztendlich zu einem nachweisbaren Signal, beispielsweise zu einem Farbumschlag führt.

Der Reagenzträger 10 und die Reagenzschicht 7 können aus dem gleichen oder unterschiedlichen Trägermaterial bestehen. Prinzipiell ist das Material des Reagenzträgers 10 aber aus dem gleichen Stoffangebot ausgewählt das sich auch für die Reagenzschicht 7 eignet.

Überraschenderweise bildet sich im zusammengedrückten Zustand des Testträgers 1 innerhalb der Schichten 7, 3 und 10 sehr schnell eine homogene Lösung, so daß das nachweisbare Signal in einer Lösung, deren Schichtdicke der Summe der Dicke sämtlicher Schichten zwischen Klappe 9 und Basisfolie 2 entspricht, bestimmt werden kann. Diese Bestimmung erfolgt vorzugsweise apparativ und ist aufgrund der relativ großen Schichtdicke sehr genau. Überraschenderweise hat sich gezeigt, daß durch die Tatsache, daß die flüssigkeitsabsorbierende Schicht verhältnismäßig dick und bevorzugt aus einem faserigen Material gebildet ist, die Qualität einer optischen Messung nicht beeinträchtigt wird. Es ergibt sich sogar eine besonders gute Meßgenauigkeit, wenn die flüssigkeitsabsorbierende Schicht 3 aus einem Material mit einem verhältnismäßig geringen optischen Absorptionsvermögen hergestellt ist, so daß sie vom Meßlicht eines entsprechenden Remissionsphotometers durchdrungen wird. Wenn auch das Material der Schicht 7 und des Trägers 10 solche optischen Eigenschaften aufweisen, muß entweder die Klappe 9 oder die Basisfolie 2, bevorzugt aber die Basisfolie 2 diffus reflektierend sein. Dadurch wird das Meßlicht durch die Schichten zurückreflektiert und von dem Meßempfänger des Reflektionsphotometers nachgewiesen. Dadurch, daß das Meßlicht die volle Schichtstärke der Schichten 3, 7 und 10 zweimal durchdringt, ist die der Messung zugrundeliegende optische Schichtdicke verhältnismäßig groß, was zu einer ausgezeichneten Empfindlichkeit der Messung führt.

Während die spezielle Ausführungsform der Fig. 1 jeweils nur eine Reagenzschicht 7 in der Transportzone

EP 0 309 883 B1

3 b und nur einen Reagenzträger 10 unter der Klappe 9 aufweist, sei darauf hingewiesen, daß es unter Umständen selbstverständlich auch vorteilhaft sein kann, mehrere Reagenzschichten 7 über- oder nebeneinander zwischen Tragschicht und flüssigkeitsabsorbierender Schicht oder eine oder mehrere Schichten über und eine oder mehrere Schichten unter der flüssigkeitsabsorbierenden Schicht anzuordnen. Ebenso ist es auch möglich, Reagenzschichten sowohl in der Probenauftragszone als auch in der Transportzone anzubringen. So ist es z. B. denkbar, zur Erleichterung der Erythrozytenabtrennung über der Erleichterung der Erythrozytenabtrennung über der Plasmaabtrennschicht 4, z. B. aus Glasfaservlies, unter dem Abdecknetz 5 eine Reagenzschicht anzuordnen, die mit Erythrozytenabtrennsubstraten nach DE-OS 33 23 973 imprägniert ist und gleichzeitig Reagenzien in Reagenzschicht 7 zwischen Tragschicht und flüssigkeitsabsorbierender Schicht einzusetzen.

Ebenso kann es auch von Vorteil sein, mehrere Reagenzträger 10 unter der Klappe 9 einzusetzen oder aber auch zusätzlich zwischen Reagenzträger 10 und Klappe 9 eine Reagenzschicht auf die Klappe 9 aufzubringen, z. B. in Form eines Films.

Im Falle, daß es nicht notwendig ist, die für die Bestimmungsreaktion erforderlichen Schritte zeitlich definiert zu entkoppeln, ist es selbstverständlich, daß auf eine Klappe 9 und auf solche Reagenzschichten verzichtet werden kann, die erst durch externe Manipulation zu einem gegebenen Zeitpunkt mit der flüssigkeitsabsorbierenden Schicht in Verbindung gebracht werden. Alle für den Test erforderlichen Reagenzien können dann wie vorstehend beschrieben so angeordnet sein, daß sie mit der flüssigkeitsabsorbierenden Schicht dauerhaft in Verbindung stehen.

Mit dieser Auswahl von Möglichkeiten soll angedeutet werden, daß es je nach durchzuführendem Test naheliegende Gründe geben kann, das vorgestellte Grundprinzip des erfindungsgemäßen Testträgers zu variieren, ohne damit dessen vorteilhafte Wirkungen entscheidend zu verändern.

Im nachfolgenden wird die Erfindung durch Beispiele näher erläutert, ohne daß diese eine Einschränkung des Anmeldungsgegenstandes bedeuten sollen.

Beispiel 1

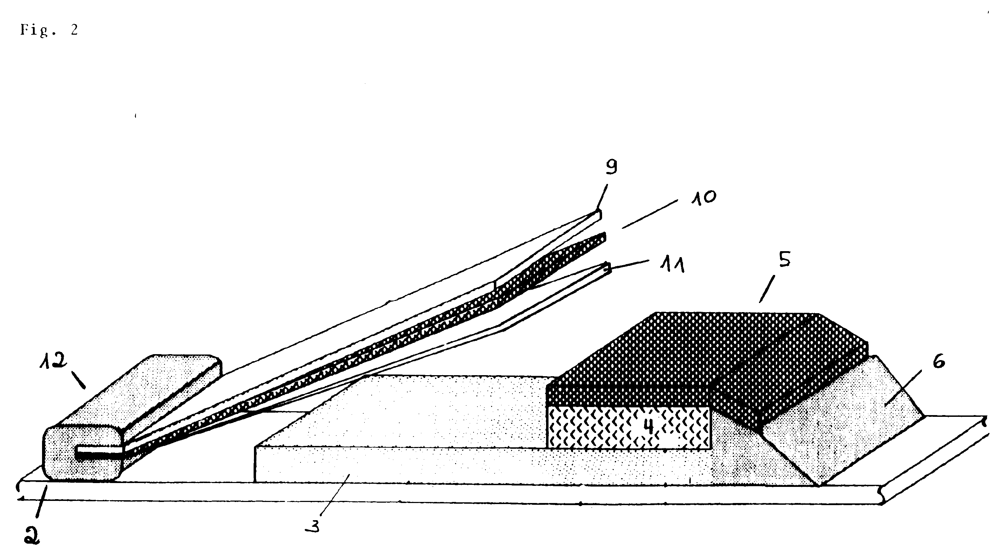

Testträger zur Bestimmung von Bilirubin in Blut entsprechend Fig. 2
a) Herstellung des Reagenzträgers 11
260 g/l Dyphyllin in Wasser werden auf Teebeutelpapier 212 (Schoeller und Hoesch, Gernsbach, BRD) getränkt und 10 Minuten bei 50 °C getrocknet.
b) Herstellung des Reagenzträgers 10
9 g/l 2-Methoxy-4-nitrophenyldiazonium-tetrafluoroborat in Wasser werden auf Teebeutelpapier 212 getränkt und 10 Minuten bei 50 °C getrocknet.

Mit den Reagenzträgern 10 und 11 werden Teststreifen entsprechend Fig. 2 hergestellt. Reagenzträger 10 und 11 werden zusammen mit einer 0,2 mm dicken Deckfolie 9 aus Polycarbonat (Pokalon, Firma Lonza, Weil, BRD) über eine Klebestelle 12 auf einer Basisfolie 2 befestigt, auf der bereits ein 15 mm breites Glasfaservlies 3 (Dicke 0,25 mm, Flächengewicht ca. 25 g/m$^2$) aufgebracht ist, so daß das freie Ende der Träger 10 und 11 sowie der Klappe 9 noch 6 mm über das Vlies reicht. Auf dem Glasfaservlies 3 ist eine Plasmaabtrennschicht 4 ebenfalls aus Glasfaservlies angebracht, welche beide durch ein Nylonschutznetz 5 mittels eines Schmelzklebestreifens 6 mit der Basisfolie 2 verbunden sind.

Die Funktion der Streifen wird durch Messung bilirubinhaltiger Proben mit Hilfe des Gerätes "Reflotron" (Boehringer Mannheim GmbH, Mannheim, BRD) vorgenommen. Die Auswertung erfolgt über empirisch ermittelte Funktionskurven.

Meßergebnisse:

I. Unbefriedigende Dyphyllinwirkung, da keine echte zeitlich entkoppelte Vorreaktion und keine vollständige Freisetzung des gebundenen Bilirubins in der Probe erfolgt.
Zur Referenz werden Nativseren gemessen. Die Reflotronmessungen erfolgen mit Vollblut.

7

| Soll Naßreferenzmethode | Messung Reflotron | Differenz zu Soll |
|---|---|---|
| mg/dl | n = 10   mg/dl | % |
| 0,8 | 0,6 | 25 |
| 1,2 | 0,9 | 25 |
| 1,4 | 0,7 | 50 |
| 1,8 | 1,3 | 28 |
| 2,5 | 1,4 | 44 |

II. Abhängigkeit der Meßergebnisse vom Dosiervolumen.

Eine aufgestrockte Kontrollprobe mit freiem Bilirubin besitzt einen Sollwert von 5,2 mg/dl. Es wurden je 10 Meßwerte genommen.

| Dosiervolumen ($\mu$l) | 26 | 28 | 30 | 32 | 34 |
|---|---|---|---|---|---|
| Meßwert (mg/dl) | 2,5 | 4,1 | 5,2 | 5,2 | 5,4 |
| vK (%) | 20,7 | 8,3 | 3,5 | 3,8 | 4,2 |

Beispiel 2

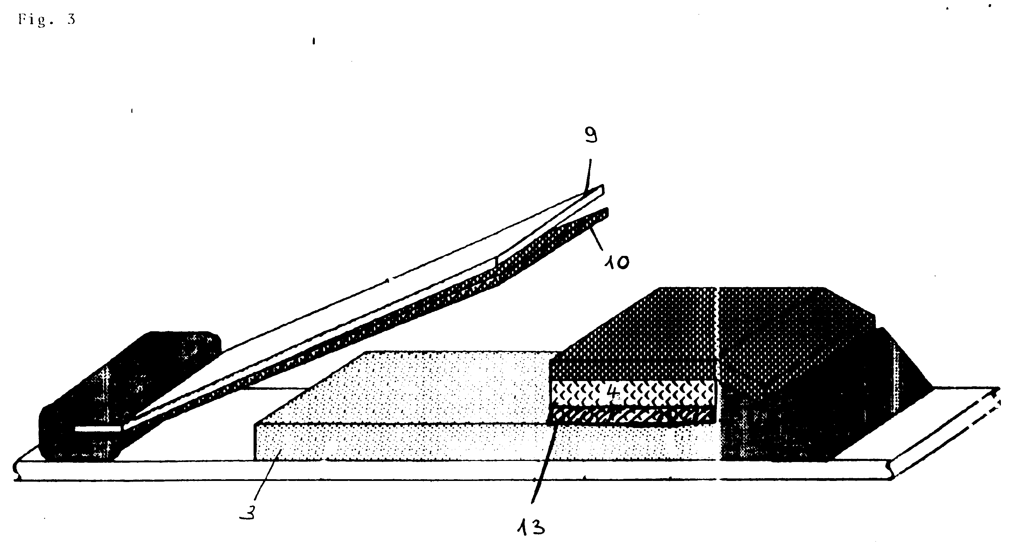

Testträger zur Bestimmung von Bilirubin in Blut entsprechend Fig. 3

Es werden unter Verwendung der Reagenzträger aus Beispiel 1 a) und b) Teststreifen entsprechend Fig. 3 hergestellt.

Das Diazoniumpapier 10 wird unter der Klappe 9 angebracht, das Dyphyllinpapier 13 (das dem in Beispiel 1 hergestellten Träger 11 entspricht) zwischen die beiden Glasfaservliese 3 und 4. Die Herstellung des Testträgers in Fig. 3 erfolgt analog dem in Beispiel 1 beschriebenen.

Die Meßergebnisse mit Serum und Plasma sind befriedigend.

Führt man die Analyse mit Blut durch, tritt je nach Probe unterschiedlich starke Hämolyse auf. Es gelangt Hämoglobin unter das Meßauge, wird vom Gerät gemessen und täuscht zu hohe Bilirubinwerte vor. Bei einer typischen Blutprobe werden folgende Werte gemessen:

Plasma 3,2 mg/dl

Blut 8,5 mg/dl

Beispiel 3

Erfindungsgemäßer Testträger zur Bestimmung von Bilirubin in Blut entsprechend Fig. 1

Es werden unter Verwendung der Testträger aus Beispiel 1 a) und b) Teststreifen entsprechend Fig. 1 hergestellt.

Das Diazoniumpapier 10 befindet sich unter der Klappe 9, das Dyphyllinpapier 7 ist unter dem Glasfaservlies 3 angebracht. Die Herstellung des Testträgers in Fig. 1 erfolgt analog dem in Beispiel 1 beschrieben.

Meßergebnisse

I. Ausreichende Dyphyllin-Wirkung

Klinische Prüfungen ergaben eine ausgezeichnete Übereinstimmung zwischen der Naßreferenzmethode DPD in Nativserum (Boehringer Mannheim) und dem Bilirubintest aus Vollblut mit einem Testträger gemäß Fig. 1.

28 Seren

x      = DPD-Wert

y      = Bilirubin-Wert mit erfindungsgemäßem Testträger

y    = - 0,046 + 1,01x; r = 0,999

II. Keine Hämolyse

Blut und sein abgetrenntes Plasma wurden mit dem Gerät Reflotron$^R$ (Boehringer Mannheim GmbH, Mannheim, BRD) gemessen

|        | Probe 1 | Probe 2 | Probe 3 |       |
|--------|---------|---------|---------|-------|
| Blut   | 0,72    | 1,30    | 4,53    |       |
| Plasma | 0,70    | 1,28    | 4,59    | mg/dl |

III. Keine Volumenprobleme innerhalb der üblichen Dosiergrenzen von 28 bis 32 μl.

Messung mit der Kontrollprobe aus Beispiel 1 (Sollwert 5,2 mg Bilirubin/dl):

| Dosiervolumen (μl) | 26  | 28  | 30  | 32  | 34  |
|--------------------|-----|-----|-----|-----|-----|
| Meßwert (mg/dl)    | 3,8 | 4,9 | 5,2 | 5,1 | 5,3 |
| vK (%)             | 8,6 | 4,5 | 3,2 | 2,9 | 3,5 |

**Patentansprüche**

1.  Testträger zur analytischen Bestimmung eines Bestandteiles einer flüssigen Probe, insbesondere einer Körperflüssigkeit, mit einer Tragschicht, die teilweise von einer flüssigkeitsabsorbierenden Schicht, welche eine Probenauftragszone und eine Transportzone beinhaltet, bedeckt ist und einer Reagenzschicht, die mit der flüssigkeitsabsorbierenden Schicht dauerhaft in Verbindung steht, und einer oder mehreren weiteren Reagenzschichten, die durch externe Manipulation mit der flüssigkeitsabsorbierenden Schicht in Verbindung gebracht werden können,
dadurch gekennzeichnet,
daß die mit der flüssigkeitsabsorbierenden Schicht in Verbindung stehende Reagenzschicht zwischen Tragschicht und flüssigkeitsabsorbierender Schicht angeordnet ist, wobei sie an die Tragschicht fixiert ist und mit der flüssigkeitsabsorbierenden Schicht vollflächig und dauerhaft in Verbindung steht und nicht mit der Probenaufgabezone überlappt
und
daß die weitere oder weiteren Reagenzschichten bei Kontakt mit der flüssigkeitsabsorbierenden Schicht die erste Reagenzschicht überdecken.

2.  Testträger gemäß Anspruch 1 dadurch gekennzeichnet, daß die Reagenzschicht mittels Schmelzkleber auf der Tragschicht befestigt ist.

3.  Testträger gemäß Anspruch 1 oder 2, dadurch gekennzeichnet, daß die mit der flüssigkeitsabsorbierenden Schicht in Verbindung stehende Reagenzschicht hämolysierend wirken kann.

4.  Verwendung eines Testträgers gemäß Anspruch 1 zur Bestimmung eines Bestandteils einer flüssigen Probe, insbesondere einer Körperflüssigkeit.

**Claims**

1.  Test carrier for the analytical determination of a component of a liquid sample, especially of a body liquid, with a carrier layer which is partly covered by a liquid-absorbing layer which contains a sample application zone and a transport zone, and a reagent layer which stands in permanent contact with the liquid-absorbing layer and one or more further reagent layers which, by external manipulation, can be brought into contact with the liquid-absorbing layer, characterised in that the reagent layer standing in contact with the liquid-absorbing layer is arranged between carrier layer and liquid-absorbing layer, whereby it is fixed on the carrier layer and stands in contact full-facedly and permanently with the liquid-absorbing layer and does not overlap with the sample application zone and that the further reagent layer or layers cover the first reagent layer in the case of contact with the liquid-absorbing layer.

2. Test carrier according to claim 1, characterised in that the reagent layer is fixed to the carrier layer by means of melt adhesive.

3. Test carrier according to claim 1 or 2, characterised in that the reagent layer standing in contact with the liquid-absorbing layer can act haemolytically.

4. Use of a test carrier according to claim 1 for the determination of a component of a liquid sample, especially of a body fluid.

**Revendications**

1. Support de test pour la détermination analytique d'un constituant d'un échantillon liquide, en particulier d'un liquide corporel, comprenant une couche de support qui est partiellement recouverte par une couche absorbante de liquide, qui comporte une zone d'application de l'échantillon et une zone de transport, et une couche de réactif qui est en contact durable avec la couche absorbante de liquide, et
   une ou plusieurs autres couches de réactif, qui peuvent être mises en contact avec la couche absorbante de liquide par manipulation externe,
   caractérisé en ce que la couche de réactif en contact avec la couche absorbante de liquide est disposée entre la couche de support et la couche absorbante de liquide, en étant fixée sur la couche de support et en étant en contact durable avec la couche absorbante de liquide sur toute sa surface et sans pénétrer dans la zone d'application de l'échantillon,
   et en ce que la ou les autres couches de réactif, lorsqu'elles entrent en contact avec la couche absorbante de liquide, recouvrent la première couche de réactif.

2. Support de test selon la revendication 1, caractérisé en ce que la couche de réactif est fixée sur la couche de support au moyen de colle à fusion.

3. Support de test selon la revendication 1 ou 2, caractérisé en ce que la couche de réactif en contact avec la couche absorbante de liquide peut avoir une action hémolytique.

4. Utilisation d'un support de test selon la revendication 1, pour la détermination d'un constituant d'un échantillon liquide, en particulier d'un liquide corporel.

Fig. 1

EP 0 309 883 B1

Fig. 2

EP 0 309 883 B1

Fig. 3

EP 0 309 883 B1